# EUROPEAN PATENT APPLICATION

(11) **EP 4 588 930 A1**
(43) Date of publication of application: **23.07.2025**
(21) Application number: 23865379.4
(22) Date of filing: 06.09.2023
(51) Int. Cl.: C07H 17/065, C07H 15/203, C07B 61/00

(54) **METHOD FOR PRODUCING GLYCOSIDE**

(30) Priority: 16.09.2022 JP 2022148338
(71) Applicant: Resonac Corporation, Tokyo 105-7325 (JP)
(72) Inventor: ISHIBASHI, Yoshitaka, Tokyo 105-7325 (JP); MIYATA, Toko, Tokyo 105-7325 (JP)
(74) Representative: Strehl & Partner mbB
(86) International application number: PCT/JP2023/032509
(87) International publication number: WO 2024/058024

(57) **Abstract**

Provided is method for producing a glycoside in which a phenolic compound is bonded to an anomeric position of a sugar, the method including: a step (1) of reacting a peracetylated sugar with the phenolic compound in the presence of an acid catalyst; and a step (2) of directly adding a deacetylating agent and a deacetylating solvent selected from the group consisting of water and an alcohol to a reaction mixture obtained in the step (1) to perform a deacetylation reaction.

## Description

### TECHNICAL FIELD

The present invention relates to a method for producing a glycoside in which a phenolic compound is bonded to an anomeric position of a sugar. Glycosides are useful as pharmaceuticals, agricultural chemicals, cosmetics, reagents, and the like.

Priority is claimed on Japanese Patent Application No. 2022-148338, filed September 16, 2022, the content of which is incorporated herein by reference.

### BACKGROUND ART

One of compounds synthesized as a glycoside is tocopheryl glucoside in which glucose is bonded to vitamin E. Tocopheryl glucoside is used as a derivative of tocopherol having various pharmacological activities such as a peripheral vasodilator action and a histamine release inhibitory action.

A glycoside is usually synthesized by bonding an alcohol or a phenolic compound to an anomeric position (1-position of a pyranose ring or a furanose ring) of a sugar in which the hydrogen of a hydroxyl group is substituted with another substituent (for example, an acetyl group) via an ether bond, and converting a substituent on a sugar skeleton into a hydroxyl group.

Patent Documents 1 to 3 disclose synthesis examples in which tocopheryl glycoside is synthesized through an intermediate synthesized by glycosidation of a peracetylated sugar and vitamin E.

In Patent Documents 1 and 2, a reaction for generating an intermediate is carried out using a peracetylated sugar and vitamin E as raw materials and using an acid catalyst such as p-toluenesulfonic acid, ferric chloride, cupric chloride, zinc chloride, aluminum chloride, boron trifluoride, titanium tetrachloride, or tin tetrachloride. **In** Patent Documents 1 and 2, after the reaction between the peracetylated sugar and vitamin E, an intermediate is isolated by an isolation operation such as column chromatography, and the isolated intermediate is subjected to a deacetylation reaction to obtain tocopheryl glucoside. The methods described in Patent Documents 1 and 2 are not industrially suitable production methods because the isolation operation of the intermediate is complicated.

Patent Document 3 describes that, as a catalyst for a glycosidation reaction, a main catalyst selected from methanesulfonic acid, p-toluenesulfonic acid, sulfuric acid, ferric chloride, titanium tetrachloride, tin tetrachloride, and the like, and a co-catalyst which is a phase transfer catalyst are used. In the synthesis example described in Patent Document 3, different solvents are used in a glycosidation step of reacting a peracetylated sugar with a phenolic compound in the presence of a catalyst and in a deacetylation step of deacetylating an intermediate obtained in the glycosidation step. In this method, it is necessary to perform solvent replacement after the glycosidation step.

### Citation List

### Patent Documents

Patent Document 1: Japanese Unexamined Patent Application, First Publication No. H2-144151
Patent Document 2: Japanese Examined Patent Application, Second Publication No. H1-55278
Patent Document 3: Japanese Unexamined Patent Application, First Publication No. 2008-133275

### SUMMARY OF INVENTION

### Technical Problem

In the methods described in Patent Documents 1 to 3, it is necessary to perform operations such as isolation and purification of an intermediate and/or solvent replacement after the glycosidation reaction, and thus these methods are not suitable for industrially producing a glycoside. Therefore, there is a demand for a simpler method for producing a glycoside which is suitable for industrial use.

Therefore, an object of the present invention is to provide a simple method for producing a glycoside.

### Solution to Problem

The present invention includes the following aspects.
[1] A method for producing a glycoside in which a phenolic compound is bonded to an anomeric position of a sugar, the method including: a step (1) of reacting a peracetylated sugar with the phenolic compound in a presence of an acid catalyst; and a step (2) of directly adding a deacetylating agent and a deacetylating solvent selected from the group consisting of water and an alcohol to a reaction mixture obtained in the step (1) to perform a deacetylation reaction.
[2] The method for producing a glycoside according to [1], in which the phenolic compound is vitamin E.
[3] The method for producing a glycoside according to [1] or [2], in which the acid catalyst is at least one catalyst selected from the group consisting of trifluoromethanesulfonic acid and copper (II) trifluoromethanesulfonate.

### Advantageous Effects of Invention

According to the present invention, it is possible to provide a simple method for producing a glycoside.

### DESCRIPTION OF EMBODIMENTS

Hereinafter, embodiments according to the present invention will be described in detail. However, the present invention is not limited to the embodiments described below.

### (Method for producing glycoside)

In one embodiment, the present invention provides a method for producing a glycoside in which a phenolic compound is bonded to an anomeric position of a sugar. The production method of the present embodiment includes a step (1) of reacting a peracetylated sugar with the phenolic compound in the presence of an acid catalyst and a step (2) of directly adding a deacetylating agent and a deacetylating solvent selected from the group consisting of water and an alcohol to a reaction mixture obtained in the step (1) to perform a deacetylation reaction.

In the production method according to the present embodiment, the reaction mixture obtained in the step (1) (hereinafter, also referred to as "reaction mixture (1)") is directly provided for the step (2). That is, the step (2) is carried out after the step (1) without separately carrying out steps such as removal and extraction of the components contained in the reaction mixture (1).

### [Step (1)]

**In** the step (1), the peracetylated sugar and the phenolic compound react with each other in the presence of an acid catalyst.

### <Peracetylated sugar>

The term "peracetylated sugar" refers to a sugar in which all the hydroxyl groups of the sugar are protected by an acetyl group. Examples of the peracetylated sugar include peracetylated sugars of hexoses such as glucose, mannose, galactose, fucose, and rhamnose; and peracetylated sugars of pentoses such as ribose and arabinose.

Specific examples of the peracetylated sugar include penta-O-acetyl-β-D-glucopyranose (pentaacetyl-β-D-glucose), penta-O-acetyl-α-D-glucopyranose (pentaacetyl-α-D-glucose), 1,2,3,4,6-penta-O-acetyl-D-mannopyranose, penta-O-acetyl-β-D-galactopyranose, and 1,2,3,4-tetra-O-acetyl-α-L-fucopyranose. As the peracetylated sugar, pentaacetyl-β-D-glucose or pentaacetyl-α-D-glucose is preferable, and penta-O-acetyl-β-D-glucose is more preferable.

The peracetylated sugar can be produced by acetylating a desired sugar by a known acetylation method. Examples of a known acetylation method include a method using acetic anhydride, sodium acetate, and acetyl chloride.

The amount of the peracetylated sugar to be used in the step (1) is preferably 1.0 to 1.6 molar equivalents with respect to the amount of the phenolic compound to be used. It is preferable to use an excess amount of the peracetylated sugar with respect to the phenolic compound.

### <Phenolic compound>

The term "phenolic compound" refers to a compound having a phenol skeleton. The phenol skeleton is a structure in which at least one hydrogen atom of a benzene ring is substituted with a hydroxyl group. The benzene ring in the phenol skeleton may form a fused ring with another aromatic ring or aliphatic ring. The phenolic compound is a compound selected from phenols. The phenolic compound is not particularly limited, and a known phenolic compound can be used. Examples of the phenolic compound include phenol, catechol, cresol, methoxyphenol, bromophenol, xylenol, ethylphenol, naphthol, resorcinol, hydroquinone, salicyl alcohol, and vitamin E. As the phenolic compound, vitamin E is preferable.

Vitamin E is tocopherol and tocotrienol. The tocopherol is a compound represented by Formula (1). The tocotrienol is a compound represented by Formula (2). 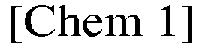 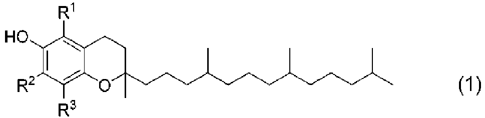 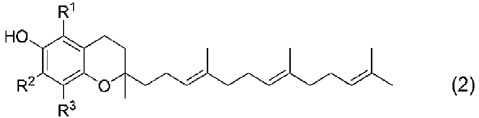 (In the formulae, R¹, R², and R³ each independently represent a hydrogen atom or a methyl group.)

The tocopherol and the tocotrienol are present as α-tocopherol and α-tocotrienol (R¹, R², and R³ = CH₃), β-tocopherol and β-tocotrienol (R¹ and R³ = CH₃, R² = H), γ-tocopherol and γ-tocotrienol (R¹ = H, R² and R³ = CH₃), and δ-tocopherol and δ-tocotrienol (R¹ and R² = CH₃, R³ = H) depending on R¹, R², and R³ in General Formulae (1) and (2).

In the case where vitamin E is used as the phenolic compound, any of α-tocopherol, β-tocopherol, γ-tocopherol, δ-tocopherol, α-tocotrienol, β-tocotrienol, γ-tocotrienol, or δ-tocotrienol may be used.

Since each of tocopherol and tocotrienol has an asymmetric carbon atom at the 2-position of the chroman ring, stereoisomers of a d form and an 1 form and a dl form are present. Each of the tocopherol and the tocotrienol may be any of these stereoisomers thereof.

### <Acid catalyst>

Examples of the acid catalyst used in the step (1) include mineral acids such as sulfuric acid, phosphoric acid, and heteropoly acid; and organic acids such as a boron trifluoride diethyl ether complex, methanesulfonic acid, trifluoromethanesulfonic acid, p-toluenesulfonic acid, trifluoroacetic acid, trifluoromethanesulfonic acid, and copper (II) trifluoromethanesulfonate.

Among these, from the viewpoint of improving the yield, the acid catalyst is preferably at least one catalyst selected from the group consisting of trifluoromethanesulfonic acid and copper (II) trifluoromethanesulfonate. As the acid catalyst, any one of trifluoromethanesulfonic acid or copper (II) trifluoromethanesulfonate may be used alone, or a mixture of trifluoromethanesulfonic acid and copper (II) trifluoromethanesulfonate may be used.

The amount of the acid catalyst to be used in the step (1) is preferably 1 to 10 mol and more preferably 1.5 to 3 mol with respect to 100 mol of the phenolic compound.

In a reaction in which an acid catalyst is used as a catalyst, the hydroxyl group of the phenolic compound attacks the anomeric position of the peracetylated sugar, and the acetyl group that protects the hydroxyl group at the anomeric position is replaced with the phenolic compound. **In** the peracetylated sugar, since the hydroxyl group at the 2-position of the sugar is protected by an acetyl group, a 1,2-trans-glycoside derivative is preferentially obtained by the involvement of adjacent groups.

### <Reaction>

Examples of the solvent used in the step (1) include toluene, benzene, ethylbenzene, xylene, ethyl acetate, methyl acetate, and methylcyclohexane. As the solvent, toluene, xylene, ethylbenzene, or benzene is preferable, and toluene is more preferable.

The reaction temperature and the reaction time can be suitably adjusted depending on the kind of the solvent and the catalyst used. The reaction temperature and the reaction time may be determined according to the progress of the reaction. The target reaction can usually be achieved by carrying out the reaction at about 0°C to 100°C for 0.5 to 24 hours under normal pressure. The reaction temperature may be, for example, 10°C to 80°C, 20°C to 70°C, 20°C to 60°C, 20°C to 50°C, or 20°C to 40°C. In the step (1), the reaction can proceed under relatively mild temperature conditions by using trifluoromethanesulfonic acid or copper (II) trifluoromethanesulfonate as a catalyst.

The reaction time depends on the amount of the reactant, but may be, for example, 1 to 25 hours, 1 to 15 hours, 1 to 10 hours, 1 to 5 hours, or 2 to 5 hours.

A glycoside derivative (hereinafter, also referred to as "acetylated glycoside derivative") in which the phenolic compound is bonded to the anomeric position of the sugar and the hydroxyl group is acetylated can be obtained by the reaction between the peracetylated sugar and the phenolic compound. The glycosidic bond formed by the reaction between the sugar and the phenolic compound may be an α-glycosidic bond or a β-glycosidic bond. According to the production method of the present embodiment, a glucoside derivative in which a glycosidic bond and an acetyl group at the 2-position are in a trans relationship can be obtained with satisfactory selectivity. From the viewpoint that the product is not a mixture with a glucoside derivative that is in a cis relationship, an isolation operation of the product, which is necessary in some cases, is not required. The production method according to the present embodiment is excellent in such a viewpoint in addition to the viewpoint of being simple. In the case where the phenolic compound is vitamin E, a bond between the sugar and -O- at the 6-position of the chroman ring of vitamin E is in a trans relationship with the hydroxyl group at the 2-position of the sugar. The reason why the glucoside derivative which is in a trans relationship is obtained with satisfactory selectivity is due to the effect of the involvement of the adjacent groups of the acetyl group which is the protective group at the 2-position.

As an example, the reaction of the peracetylated glucose with the phenolic compound is shown in Reaction Formula (I). An acetylated β-glucoside derivative in which the phenolic compound is bonded to the anomeric position of the peracetylated glucose can be obtained by the following reaction. 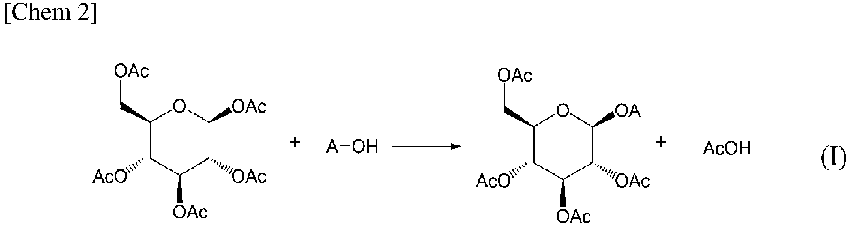 (In the formula, Ac represents an acetyl group. A-OH represents a phenolic compound, and A represents an atomic group other than a hydroxyl group.)

### [Step (2)]

In the step (2), the reaction mixture (1) obtained in the step (1), a deacetylating agent, and a deacetylating solvent are directly added to perform a deacetylation reaction. That is, other steps such as a purification step such as extraction or distillation are not interposed between the step (1) and the step (2).

### <Deacetylating agent>

The deacetylation reaction in the step (2) can be started by adding a deacetylating agent to the reaction mixture (1). The term "deacetylating agent" refers to a compound that catalyzes a reaction of deacetylating a hydroxyl group protected by an acetyl group. Examples of the deacetylating agent include a base catalyst. Examples of the base catalyst that can be used as the deacetylating agent include an alkali metal hydroxide (sodium hydroxide, potassium hydroxide, or the like) and an alkali metal alkoxide (sodium methoxide and the like), but the present invention is not limited thereto. As the deacetylating agent, sodium hydroxide or sodium methoxide is preferable, and sodium methoxide is more preferable.

### <Deacetylating solvent>

In the step (2), the deacetylating agent is added to the reaction mixture (1) together with the deacetylating solvent. **In** this case, the deacetylating agent may be dissolved in a deacetylating solvent and then added to the reaction mixture (1). The term "deacetylating solvent" refers to a solvent used in the deacetylation reaction, and is used together with a deacetylating agent. The deacetylating solvent can be suitably selected depending on the kind of the deacetylating agent. The deacetylating solvent is at least one selected from the group consisting of water and an alcohol. As the alcohol, an alkanol (alkyl alcohol) having 1 to 10 carbon atoms is preferable, and methanol is more preferable. Examples of the combination of the deacetylating agent and the deacetylating solvent include a combination of sodium hydroxide and water; and a combination of sodium methoxide and methanol. Specific examples of the solution (deacetylating agent solution) obtained by dissolving the deacetylating agent in a deacetylating solvent include a sodium hydroxide aqueous solution and a sodium methoxide-methanol solution.

The amount of the deacetylating agent to be added to the reaction mixture (1) may be any amount as long as the acetylated glycoside derivative generated in the step (1) can be deacetylated. The amount of the deacetylating agent to be added can be set according to the kind of the deacetylating agent and the kind of the deacetylating solvent.

In the case where alkali metal alkoxide (for example, sodium methoxide) and an alcohol (for example, methanol) are used as the deacetylating agent and the deacetylating solvent, the acetyl group on the sugar skeleton is converted into a hydroxyl group by a transesterification reaction with the alcohol. In this case, the alkali metal alkoxide acts as a catalyst for the transesterification reaction. **In** the transesterification reaction, the alkali metal alkoxide may be present in the amount of the catalyst. In the production method of the present embodiment, the step (2) is performed using the reaction mixture (1) obtained in the step (1). In the reaction mixture (1), acetic acid generated by the glycosidation reaction in the step (1) is present, and the acid used as the acid catalyst in the step (1) is also present. Therefore, the amount of the alkali metal alkoxide to be added can be set to an amount sufficient to neutralize the acetic acid and the acid catalyst and to catalyze the transesterification reaction. The amount of the alkali metal alkoxide to be added is preferably an amount more than 1 molar equivalent with respect to the phenolic compound, and for example, can be set to 1.1 molar equivalents or more, 1.2 molar equivalents or more, or 1.3 molar equivalents or more with respect to the amount of the phenolic compound to be used in the step (1). The upper limit of the amount of the alkali metal alkoxide to be added is not particularly limited, but for example, can be set to 1.6 molar equivalents or less with respect to the amount of the phenolic compound to be used in the step (1).

The amount of alcohol to be added can be set to the amount in which the acetyl group of the acetylated glycoside derivative generated in the step (1) can be deprotected by the transesterification reaction. It is preferable that an excess amount of the alcohol is added to the acetyl group contained in the acetylated glycoside derivative. The amount of the alcohol to be added is, for example, preferably 1 molar equivalent or more with respect to the acetyl group contained in the acetylated glycoside derivative generated in the step (1). The amount of the alcohol to be added can be set to 1.2 molar equivalents or more, 1.5 molar equivalents or more, or 1.7 molar equivalents or more with respect to the acetyl group contained in the acetylated glycoside derivative. The upper limit of the amount of the alcohol to be added is not particularly limited, but for example, can be set to 3 molar equivalents or less with respect to the acetyl group contained in the peracetylated sugar.

For example, in a case where a peracetylated sugar having five acetyl groups in one molecule (for example, peracetylated glucose) is used in the step (1), the amount of the alcohol to be added is preferably 6 to 15 molar equivalents with respect to the amount of the phenolic compound to be used in the step (1).

As an example, the deacetylation reaction of the peracetylated glycoside derivative using alkali metal alkoxide and an alcohol is shown in Reaction Formula (II)-**1. In** Reaction Formula (II)-1, AcOH represents acetic acid generated in the step (1). 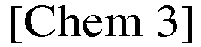 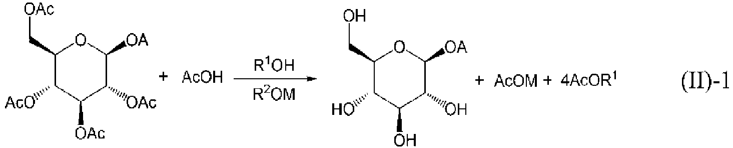 (In the formula, Ac represents an acetyl group. A represents a group derived from a phenolic compound. R¹OH represents an alcohol, and R¹ represents an alkyl group. R²OM represents alkali metal alkoxide, R² represents an alkyl group, and M represents an alkali metal.)

In the case where an alkali metal hydroxide (for example, sodium hydroxide) and water are used as the deacetylating agent and the deacetylating solvent, the hydroxide ion acts on the acetyl group to convert the acetyl group into a hydroxyl group. In the reaction mixture (1), acetic acid generated by the glycosidation reaction in the step (1) is present, and the acid used as the acid catalyst in the step (1) is also present. Therefore, the amount of the alkali metal hydroxide to be added can be set to an amount sufficient to neutralize the acetic acid and the acid catalyst and to deprotect the acetyl group contained in the acetylated glycoside derivative. It is preferable that an excess amount of the alkali metal hydroxide is added to the acetyl group contained in the acetylated glycoside derivative. The amount of the alkali metal hydroxide to be added is, for example, preferably 1 molar equivalent or more with respect to the acetyl group contained in the acetylated glycoside derivative generated in the step (1).

For example, in a case where the peracetylated sugar having five acetyl groups in one molecule (for example, peracetylated glucose) is used in the step (1), the amount of the alkali metal hydroxide to be added is preferably 5 molar equivalents or more and can be set to 20 molar equivalents or less with respect to the amount of the peracetylated sugar having five acetyl groups to be used in the step (1).

As an example, the deacetylation reaction of the peracetylated glycoside derivative using an alkali metal aqueous solution is shown in Reaction Formula (II)-2. In Reaction Formula (II)-2, AcOH represents acetic acid generated in the step (1). 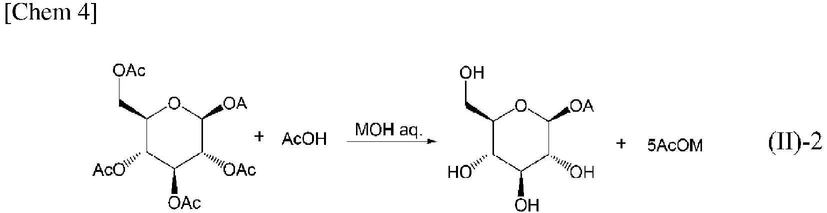 (In the formula, Ac represents an acetyl group. A represents a group derived from a phenolic compound. MOH aq. represents an alkali metal hydroxide aqueous solution, and M represents an alkali metal.)

### <Reaction>

In the production method of the present embodiment, the reaction mixture (1) obtained in the step (1) is directly provided for the step (2). The step (2) can be started by adding the deacetylating agent and the deacetylating solvent to the reaction mixture (1). The step (2) may be performed as it is in the reaction container in which the step (1) has been performed, or the reaction mixture (1) may be taken out from the reaction container in the step (1) and transferred to another reaction container to perform the step (2). In addition, a stirrer, a heating and cooling device, and the like may be suitably replaced with those used in the step (1).

No reactant other than the deacetylating agent and the deacetylating solvent is added to the reaction mixture (1). The isolation and purification operation and the solvent replacement are not performed on the reaction mixture (1) after the step (1).

It is preferable that the step (2) is started by adding only the deacetylating agent and the deacetylating solvent to the reaction mixture (1).

The deacetylation reaction of the step (2) can be promoted by adding the deacetylating agent and the deacetylating solvent to the reaction mixture (1) and maintaining an appropriate reaction temperature.

The reaction temperature and the reaction time can be suitably adjusted depending on the kind of the deacetylating agent and the kind of the deacetylating solvent. The reaction temperature and the reaction time may be determined according to the progress of the reaction. The deacetylation reaction can usually be achieved by carrying out the reaction at about 0°C to 80°C for 10 minutes to 20 hours under normal pressure. The reaction temperature may be, for example, 10°C to 70°C, 20°C to 60°C, 30°C to 60°C, 30°C to 50°C, or 30°C to 40°C.

The reaction time depends on the amount of the reactant, but may be, for example, 0.5 to 15 hours, 1 to 10 hours, 1.5 to 6 hours, 2 to 5 hours, or 2 to 4 hours.

As a specific example of the reaction condition, the reaction can be carried out at 30°C to 60°C for 10 minutes to 4 hours under normal pressure.

In the production method of the present embodiment, it is not necessary to perform an isolation operation and a purification operation, such as water washing and column chromatography, between the step (1) and the step (2). In addition, it is not necessary to carry out solvent replacement between the step (1) and the step (2). The deacetylation reaction in the step (2) can be performed by simply adding the deacetylating agent and the deacetylating solvent to the reaction mixture (1) after the step (1). Therefore, it is possible to simply and efficiently produce a glycoside in which a phenolic compound is bonded to the anomeric position of the sugar.

A glycoside in which a phenolic compound is bonded to the anomeric position of the sugar (for example, tocopheryl glycoside) has attracted attention as a raw material for pharmaceuticals, agricultural chemicals, cosmetics, reagents, and the like. According to the production method of the present embodiment, it is possible to efficiently produce a glycoside useful as a raw material for pharmaceuticals and the like.

After step (2), a glycoside in which a phenolic compound is bonded to the anomeric position can be obtained by carrying out a known purification method such as column chromatography, crystallization, decolorization with activated carbon, or recrystallization.

### Examples

Hereinafter, the present invention will be described in greater detail with examples and comparative examples, but is not limited to the following examples.

### (Analysis conditions)

A d-δ-tocopherol β-d-glucopyranoside in a reaction solution obtained by a reaction was subjected to high-performance liquid chromatography to determine a peak area, and quantified by a calibration curve created in advance. From the obtained value, the yield of d-δ-tocopheryl β-d-glucopyranoside was determined using d-δ-tocopherol as a reference. Further, as an eluent used for the analysis, a mixed solvent of methanol and water in which sodium acetate was dissolved was used.

Details of the analysis conditions are as follows.
HPLC device: Jasco
Column: Shodex ODP-50 6D × 2
Eluent: 0.1 mM CH₃COONa (MeOH/H₂O = 100/1)
Flow rate: 0.5 mL/min
Oven temperature: 40°C
Detection: ultraviolet absorbance detector (UV)

### (Example 1)

d-δ-tocopherol (Sigma-Aldrich Co., LLC, 24.8 mmol, 10 g), pentaacetyl-β-D-glucose (Tokyo Chemical Industry Co., Ltd., 24.8 mmol, 9.68 g), 4-toluenesulfonic acid monohydrate (hereinafter, also referred to as "p-TsOH") (JUNSEI CHEMICAL CO., LTD., 1.24 mmol, 0.236 g), and toluene (Kanto Chemical Co., Inc., 15 ml) were placed in a one-neck flask and stirred in an external bath at 70°C for 24 hours under normal pressure. Thereafter, methanol (FUJIFILM Wako Pure Chemical Corporation, 160.42 mmol, 5.14 g) and a 28% sodium methoxide-methanol solution (JUNSEI CHEMICAL CO., LTD., 6.6 g, sodium methoxide: 34.2 mmol, methanol: 148.3 mmol) were added to the obtained reaction solution, and the mixture was stirred in an external bath at 30°C for 1 hour to perform deacetylation. After completion of the reaction, the product in the reaction solution was analyzed by high-performance liquid chromatography, and the yield of d-δ-tocopheryl β-d-glucopyranoside was 60%.

### (Reference: isolation and purification of d-δ-tocopheryl β-d-glucopyranoside)

The reaction solution containing d-δ-tocopheryl β-d-glucopyranoside obtained in Example 1 was concentrated under reduced pressure and purified by column chromatography using an ethyl acetate solvent with a silica gel, thereby obtaining 7.56 g (13.4 mmol) of d-δ-tocopheryl β-d-glucopyranoside (isolation yield: 54%). 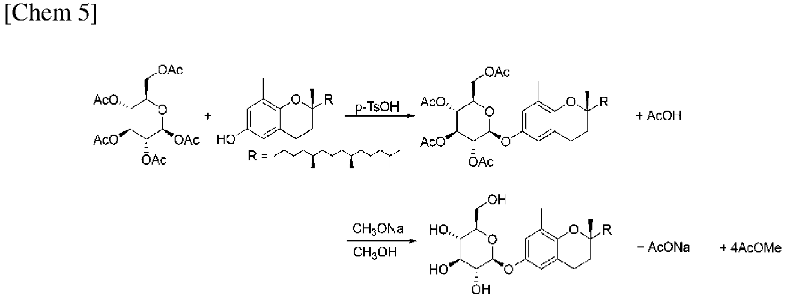 (In the formula, Ac represents an acetyl group, and Me represents a methyl group.)

### (Comparative Example 1)

Pentaacetyl-β-D-glucose (49.7 mmol, 19.4 g), d-δ-tocopherol (49.7 mmol, 20 g), p-TsOH (2.48 mmol, 0.472 g), and toluene (30 ml) were allowed to react with each other in the same manner as in Example 1. The toluene layer after the reaction was subjected to liquid separation washing with 25 g of a NaHCO₃ saturated aqueous solution three times and 25 g of distilled water once. The toluene layer was concentrated under reduced pressure and purified by column chromatography with a solvent of ethyl acetate and hexane at a mixing ratio of 1:3 using a silica gel, thereby obtaining 17.4 g (23.8 mmol) of a glycoside derivative (hereinafter, referred to as "acetylated glycoside derivative") in which tocopherol was bonded to the anomeric position and hydrogen of another hydroxyl group was acetylated.

17.4 g (23.8 mmol) of the acetylated glycoside derivative obtained above was heated and dissolved in methanol (manufactured by FUJIFILM Wako Pure Chemical Corporation, 20 ml), and the solution was placed in a one-neck flask together with a 28% sodium methoxide-methanol solution (JUNSEI CHEMICAL CO., LTD., 0.7 g), and a deacetylation reaction was carried out in an external bath at 30°C for 1 hour. After completion of the reaction, the product in the reaction solution was analyzed by high-performance liquid chromatography, and the yield of d-δ-tocopheryl β-d-glucopyranoside was 48%.

### (Reference: isolation and purification of d-δ-tocopheryl β-d-glucopyranoside)

In the case where the reaction solution containing d-δ-tocopheryl β-d-glucopyranoside obtained in Comparative Example 1 was purified by column chromatography with an ethyl acetate solvent using a silica gel, 12.07 g (21.4 mmol) of d-δ-tocopheryl β-d-glucopyranoside was obtained (isolation yield: 43%).

### (Example 2)

d-δ-tocopherol (Sigma-Aldrich Co., LLC, 29.8 mmol, 12 g), pentaacetyl-β-D-glucose (Tokyo Chemical Industry Co., Ltd., 35.9 mmol, 14 g), trifluoromethanesulfonic acid (hereinafter, also referred to as "TfOH") (Kanto Chemical Co., Inc., 0.6 mmol, 0.09 g), and toluene (Kanto Chemical Co., Inc., 45 ml) were placed in a one-neck flask and stirred in an external bath at 30°C for 3 hours under normal pressure. Thereafter, methanol (FUJIFILM Wako Pure Chemical Corporation, 106.25 mmol, 3.4 g) and a 28% sodium methoxide-methanol solution (JUNSEI CHEMICAL CO., LTD., 8.7 g, sodium methoxide: 45.1 mmol, methanol: 195.5 mmol) were added to the obtained reaction solution, and the mixture was stirred in an external bath at 30°C for 1 hour to perform deacetylation. After completion of the reaction, the product in the reaction solution was analyzed by high-performance liquid chromatography, and the yield of d-δ-tocopheryl β-d-glucopyranoside was 89%. 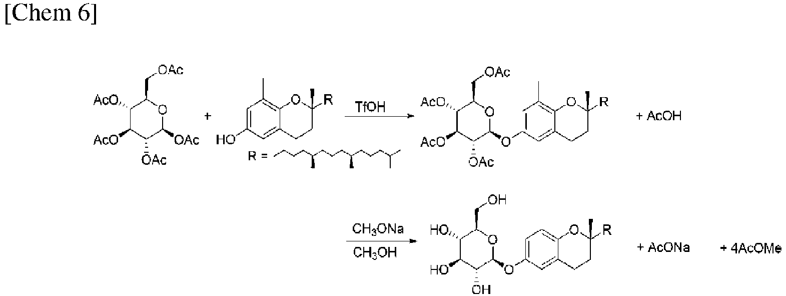 (In the formula, Ac represents an acetyl group.)

### (Example 3)

d-δ-tocopherol, pentaacetyl-β-D-glucose, TfOH, and toluene were allowed to react with each other in the same manner as in Example 2. A NaOH aqueous solution (JUNSEI CHEMICAL CO., LTD., amount of NaOH added: 310 mmol, 12.4 g) whose concentration was adjusted to 48% was added to the obtained reaction solution, and the mixture was stirred in an external bath at 30°C for 3 hours to perform deacetylation. After completion of the reaction, the product in the reaction solution was analyzed by high-performance liquid chromatography, and the yield of d-δ-tocopheryl β-d-glucopyranoside was 89%.

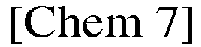

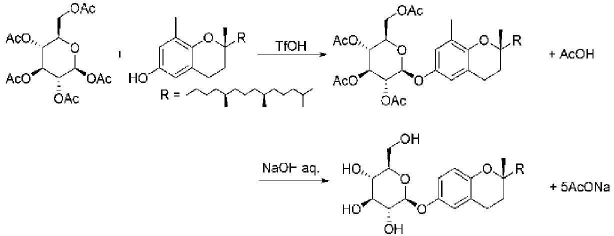

As shown in the above-described results, it was confirmed that in Examples 1 to 3, the methods were simple and the yields thereof were also improved compared with the method of Comparative Example 1.

While preferred examples of the present invention have been described and illustrated above, it should be understood that these are examples of the present invention and are not to be considered as limiting. Additions, omissions, substitutions, and other modifications can be made without departing from the scope of the present invention. Accordingly, the present invention is not limited by the description above but by the scope of the appended claims.

## Claims

1. A method for producing a glycoside in which a phenolic compound is bonded to an anomeric position of a sugar, the method comprising:
a step (1) of reacting a peracetylated sugar with the phenolic compound in a presence of an acid catalyst; and
a step (2) of directly adding a deacetylating agent and a deacetylating solvent selected from the group consisting of water and an alcohol to a reaction mixture obtained in the step (1) to perform a deacetylation reaction.

2. The method for producing a glycoside according to Claim 1,
wherein the phenolic compound is vitamin E.

3. The method for producing a glycoside according to Claim 1 or 2,
wherein the acid catalyst is at least one catalyst selected from the group consisting of trifluoromethanesulfonic acid and copper (II) trifluoromethanesulfonate.
